Europäisches Patentamt

**European Patent Office**

Office européen des brevets

(11) Publication number: **0 051 337**
**A1**

(12) # EUROPEAN PATENT APPLICATION

(21) Application number: **81201200.3**

(22) Date of filing: **29.10.81**

(51) Int. Cl.³: **C 07 D 303/36**
C 07 D 301/00, C 08 B 31/12
D 21 H 3/28

(30) Priority: **30.10.80 NL 8005953**

(43) Date of publication of application:
**12.05.82 Bulletin 82/19**

(84) Designated Contracting States:
**AT BE CH DE FR GB IT LI LU NL SE**

(71) Applicant: **CHEM-Y FABRIEK VAN CHEMISCHE
PRODUCTEN B.V.
Noordstraat 49
NL-2411 BH Bodegraven(NL)**

(72) Inventor: **Heijen, Hubertina Mathias Maria
Bockstraat 62
NL-6461 VZ Kerkrade(NL)**

(72) Inventor: **Savelkoul, Johannes Theodorus Gerardus
Schoolstraat 21
NL-6127 BE Grevenbicht(NL)**

(72) Inventor: **Smid, Jacob Kornelis
Populierenhof 33
NL-2411 SX Bodegraven(NL)**

(74) Representative: **Pinckaers, August René et al,
OCTROOIBUREAU DSM Postbus 9
NL-6160 MA Geleen(NL)**

(54) Process for the preparation of a cationization reagent and starch cationized with this reagent.

(57) The invention relates to a process for the preparation of a cationization reagent in which a glycidyltrialkylammoniumhalide is converted with the aid of sulphuric acid or a sulphate in the corresponding glycicyltrialkylammonium sulphate. The invention also relates to starch being cationized with said glycidyltrialkylammonium sulphate.

EP 0 051 337 A1

-1-

## PROCESS FOR THE PREPARATION OF A CATIONIZATION REAGENT AND STARCH CATIONIZED WITH THIS REAGENT

The invention relates to a process for the preparation of a cationization reagent and to starch cationized with this reagent.

Suitable reagents for the cationization of starch, i.e. the etherification of starch with a cation-active compound are the glycidyltrimethylammonium halides (hereafter abbreviated to gta-halides), as is known from, for instance, United States Specification 3,475,458. In practice, the gta-halide mostly used for the cationization of starch is gta-chloride.

It has appeared, however, that starch cationized with gta-chloride, although suitable for various applications, has corrosive properties, which can cause corrosion, for instance, when starch cationized with gta-chloride is applied in the paper industry, of the paper machines getting into contact with the cationized starch.

It has now been found that starch cationized with glycidyltrialkylammonium sulphate (hereafter abbreviated to gta-sulphate) is less corrosive than starch cationized with gta-chloride, and that the gta-sulphate needed for this cationization, for which no practicable preparation method had been known until now, can very suitably be obtained from a gta-halide.

The process according to the invention for the preparation of a cationization reagent is characterized in that a gta-halide is started from and converted into the corresponding gta-sulphate with the aid of sulphuric acid or a sulphate.

The convertion according to the invention of a gta-halide into a gta-sulphate is by preference carried out with the aid of an anion exchanger, in particular a strongly basic anion exchanger, in the sulphate form. To this end, various anion exchangers can be applied, for instance the exchangers commercially available under the name of Lewatit M 500 (strongly basic anion exchanger on a polystyrene basis with a quaternary ammonium group as a functional group), Lewatit MP 500 (the same as Lewatit M 500, but with a macroporous structure), Lewatit MP 62 (weakly basic anion exchanger on a polystyrene basis with a tertiary amine as functional group) and Dualite A 374 (weakly basic anion exchanger with macroporous polyacryl structure and a secondary amine as functional group). By preference, an anion exchanger is applied which has a macroporous structure (a structure involving exchanger pores with a mean diameter of more than 10 nm and/or an internal surface area of the exchanger of more than 5 $m^2$ per gramme).

The conversion with the aid of the anion exchanger can be carried out by passing an aqueous solution of the gta-halide, preferably gta-chloride, across the exchanger in the sulphate from. The anion exchangers can be converted to the sulphate form for instance passing sulphuric acid or an aqueous sulphate solution across the exchanger in the chloride form.

Different values can be chosen for the concentration of the aqueous gta-halide solution to be passed across the exchanger, for example concentrations between 5 wt.-% and the saturation concentration of the gta-halide in water. By preference, a concentration of 50-88 wt.-% is applied. The ion exchange can in addition be carried out at different temperatures, for instance at a temperature of 5-90 °C. For good performance of the ion exchanger the temperature will have to be at least so high that no solids crystallize on the exchanger. By preference, ambient temperature is applied, because then the rate of ion exchange is sufficiently high and the chance of the glycidyl group decomposing is very small.

Different trialkyl groups may be used in the gta-halide, in dependence on the trialkyl group desired in the gta-sulphate. By preference, a trimethyl group is applied as trialkyl group in the gta-halide, but other trialkyl groups can also be applied, such as, for instance, the groups triethyl, tributyl, trilauryl, dimethylhexyl, dipropyldecyl, diethylstearyl, methylethylbutyl, dimethyllauryl and dimethylstearyl.

The cationization of starch with gta-sulphate can be carried out in the same ways as the cationization with a gta-halide, for instance as described in the book 'Starch: Chemistry and Technology' by R.L. Whistler and E.F. Paschall, Chapter 16 (Academic Press 1967) and in United States Patent Specification 4,088,600. The degree of substitution of the cationized starch, i.e. the number of moles of cation-active substituent per mole of anhydroglucose units in the starch, can be varied. With a degree of substitution of the cationized starch of between 0.005 and 0.5 a good result can be achieved.

The starch cationized with gta-sulphate is suitable for various applications, for instance as a finishing agent in the textile industry and as an auxiliary in paper finishing.

The invention will be elucidated in the following examples, the trialkyl group in the gta-chloride being a trimethyl group.

## Example I (preparation of gta-sulphate)

A column with a diameter of 17 mm is filled with 14.5 ml Lewatit MP 500 in the chloride form. With the aid of an aqueous sodium sulphate solution (0.15 mol $Na_2SO_4$ per litre) the exchanger is converted to the sulphate form. Subsequently, 10 ml aqueous gta-chloride solution (23 wt.-%) is passed through the column at a rate of 2 ml per minute, after which 100 ml demineralized water is washed through.

An aqueous solution containing 2.3 wt.-% gta-sulphate is obtained. The gta-sulphate yield is virtually 100 %. The total amount of epoxide in the gta-sulphate is equal to that in the products started from.

## Example II (preparation of gta-sulphate)

In the way described in example I, 4.61 g aqueous gta-chloride solution (85 wt.-%) is converted into gta-sulphate in a column filled with 21.3 ml Lewatit MP 500. The yield is substantially 100 % and the total amount of epoxide in the gta-sulphate is equal to that in the gta-chloride.

## Example III (preparation of gta-sulphate)

A column similar to the one in example I is filled with 24.7 ml Lewatit MP 500 in the chloride form. With the aid of dilute sulphuric acid (0.15 mol $H_2SO_4$ per litre) the exchanger is converted to the sulphate form and the residual acid washed out with demineralized water. Subsequently, 10 ml aqueous gta-chloride solution (40 wt.-%) is passed through the column at a rate of 2 ml per munite, after which 200 ml demineralized water is washed through.

The gta-sulphate yield is substantially 100 %. The total amount of epoxide in the gta-sulphate is equal to that in the product started from.

Example IV (preparation of gta-sulphate)

A column similar to the one in example 1 is filled with 11.8 g Lewatit M 500 in the chloride form, dried in air. With an aqueous sodium sulphate (0,15 mol Na2SO4 per litre) the exchanger is converted to the sulphate form. Subsequently, 3.39 g aqueous gta-chloride solution (85 wt.-%) is passed through the column at a rate of 2 ml per minute, after which 60 ml demineralized water is washed through.

The gta-sulphate yield amounts to 96 %. The total amount of epoxide in the product obtained is equal to that in the product started from.

Example V (preparation of gta-sulphate)

A column similar to the one in example 1 is filled with 9.1 g Lewatit MP 62 in the chloride form, dried in air. The exchanger is then converted to the sulphate form by passing through an aqueous sodium sulphate solution (0,15 mol Na2SO4 per litre). Subsequently 3.52 g aqueous gta-chloride solution (85 wt.-%) is passed through the column a rate of 2 ml per minute, after which 90 ml demineralized water is washed through.

The sulphate yield is 86 %. The total amount of epoxide in product obtained is the same as that in the product started from.

Example VI (preparation of cationized starch)

At room temperature 737 g starch (particle size 3-30 μm, obtained from maize) containing 12 wt.-% water is disjpersed in 722 ml demineralized water. To this dispersion first 295 g aqueous gta-sulphate solution (13 wt.-%, epoxide content 0.61 meq. per gramme of solution, obtained in the way described in example II) is added and then, slowly and with adequate stirring, a solution of 10.3 g NaOH in 208 g demineralized water. The resulting suspension is then heated to 45 °C in a water batch of 45 °C, with adequate stirring. Next, the mixture is allowed to react at this temperature for 7 hours, after which the resulting slurry is neutralized to pH 6 with sulphuric acid (50 wt.-%) and is diluted with demineralized water to a solids content of 5.5 wt.-%.

The resulting cationized starch has a degree of substitution of 0.035 (determined by nitrogen analysis according to Kjeldahl).

Electrochemical investigation of an AISI 316L steel (composition 0.019 wt.-% C, 17.3 wt.-% Cr, 12.7 wt.-% Ni and 2.7 wt.-% Mo) in the resulting aqueous solution of cationized starch, with the object to detect local corrosion, showed that no local corrosion occurred.

The corrosion test was carried out by determining the log I-E curve for a specimen bar (diameter 5 mm, length 30 mm) of said steel in the aqeuous solution of cationized starch, using a scanning rate of 2 mV per second. In this test the specimen bar was used as rotating test electrode. As reference electrode a saturated calomel electrode was used, and as counter-electrode platinum sheet.

A comparative investigation of starch which had been cationized with glycidyltrimethylammonium chloride showed local corrosion to have occurred at the specimen bar.

Microscopic investigation of the specimen bars confirmed the result found by electrochemical investigation.

## CLAIMS

1. Process for the preparation of cationization reagent, characterized in that a glycidyltrialkylammonium halide is started from and converted into the corresponding glycidyltrialkylammonium sulphate with the aid of sulphuric acid or a sulphate.

2. Process according to claim 1, characterized in that the conversion is carried out with the aid of an anion exchanger in the sulphate form.

3. Process according to claim 2, characterized in that a stronly basic anion exchanger is used.

4. Process according to either of the claims 2-3, characterized in that a macroporous anion exchanger is used.

5. Process according to any one of the claims 1-4, characterized in that glycidyltrimethylammonium chloride is started from.

6. Process according to claim 1, as substantially described and elucidated in examples I-V.

7. Cationization reagent, obtained by application of the process according to any one of the foregoing claims.

8. Starch cationized with glycidyltrialkylammonium sulphate.

9. Starch cationized with glycidyltrimethylammonium sulphate.

10. Starch cationized with a cationization reagent obtained by application of the process according to any one of the claims 1-6.

11. Starch according to claim 10, as substantially described and further elucidated in example VI.

0051337

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| | CHEMICAL ABSTRACTS, vol. 80, no. 19, 13-05-1974, pages 402-403 abstract 108346h Columbus, Ohio, US & JP - A - 73 99108 (MORIROKU SHOJI CO. LTD.) 15-12-1973) * Abstract * | 1 | C 07 D 303/36 301/00 C 08 B 31/12 D 21 H 3/28 |
| | US - A - 3 853 460 (J. BALLAND) * Whole patent * | 1 | TECHNICAL FIELDS SEARCHED (Int.Cl.³) C 07 D 303/36 |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document. but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 28-01-1982 | ALLARD |

EPO Form 1503.1   06.78